## Europäisches Patentamt

## European Patent Office

## Office européen des brevets

(19)

(11) Veröffentlichungsnummer: **0 088 098**
**B1**

(12)

# EUROPÄISCHE PATENTSCHRIFT

(45) Veröffentlichungstag der Patentschrift:
16.12.87

(51) Int. Cl.⁴: **A 61 K 31/715, C 08 L 5/16**

(21) Anmeldenummer: **82902689.7**

(22) Anmeldetag: **09.09.82**

(86) Internationale Anmeldenummer:
**PCT/HU 82/00043**

(87) Internationale Veröffentlichungsnummer:
**WO 83/00809 (17.03.83 Gazette 83/07)**

(54) **KOMPOSITION UND VERFAHREN ZUR HERSTELLUNG VON IN WÄSSRIGEM MEDIUM SCHNELL ZERFALLENDEN TABLETTEN.**

(30) Priorität: **09.09.81 HU 258681**

(43) Veröffentlichungstag der Anmeldung:
**14.09.83 Patentblatt 83/37**

(45) Bekanntmachung des Hinweises auf die Patenterteilung:
**16.12.87 Patentblatt 87/51**

(84) Benannte Vertragsstaaten:
**AT CH DE FR GB LI**

(56) Entgegenhaltungen:
**CH-A-445 129**
**US-A-4 169 079**
**US-A-4 274 985**

**CHEMICAL ABSTRACTS, Band 92, Nr. 8, 25. Februar 1980, Seite 386, Zusammenfassung 64754f, Columbus, Ohio, US; - JP - A - 7986607 DIE STÄRKE, Band 21, Nr. 5, Mai 1969, Seiten 119-123, N. WIEDENHOF u.a.: "Properties of Cyclodextrins..Part III. Cyclodextrin-epichlorhydrin resins: preparation and analysis".**

(73) Patentinhaber: **CHINOIN Gyogyszer és Vegyészeti Termékek Gyára RT., To utca 1-5, H-1045 Budapest IV (HU)**

(72) Erfinder: **SZEJTLI, Jozsef, Endrödi S. u.38- 40, H-1026 Budapest (HU)**
Erfinder: **FENYVESI, Eva, Lisznyai ut 24, H-1016 Budapest (HU)**
Erfinder: **DOSA, Eva B., Erdöalja u.52, H-1037 Budapest (HU)**
Erfinder: **WAGNER, Ildiko, Sallai u.6, H-1136 Budapest (HU)**
Erfinder: **ANTAL, Balazs, Damjanich u.42, H-1071 Budapest (HU)**
Erfinder: **KALLOI, Katalin, Bertalan L. u.22, H-1111 Budapest (HU)**
Erfinder: **VIRAG, Sandor, Sallai u.29, H-1136 Budapest (HU)**

(74) Vertreter: **Lotterhos, Hans Walter, Dr.- Ing., Lichtensteinstrasse 3, D-6000 Frankfurt am Main 1 (DE)**

**Beschreibung**

Die Erfindung betrifft eine Komposition und ein Verfahren zur Herstellung von in wäßrigem Medium rasch zerfallenden Tabletten.

Die am einfachsten zu handhabenden und am meisten verbreiteten Formulierungsformen der pharmazeutischen Präparate sind bekanntlich Tabletten und Dragées (nachfolgend kurz "Tabletten" genannt). Der wesentliche Vorteil der Tabletten besteht darin, daß sich die zu verabreichende Dosis durch einfaches Abzählen der Verabreichungseinheiten leicht und in reproduzierbarer Weise abteilen läßt.

Auf Grund der Adhäsionseigenschaften und der Stärke der Wirkstoffaktivität können die Tabletten in den meisten Fällen durch Tablettieren des Wirkstoffes per se nicht hergestellt werden. Zur Herstellung von Tabletten geeigneter Größe muß der Wirkstoff gewöhnlich mit Füllstoffen "verdünnt" werden, wobei der Tablettenmasse zur Erzielung der geeigneten Fließbarkeit und geeigneten Farbe, des gewünschten Geschmackes und Reaktionsfähigkeit sowie zum Schutz des Wirkstoffes gegenüber Oxydation weitere Zusatzstoffe zugefügt werden.

Es ist sehr wichtig, daß die Tabletten im Organismus an der gewünschten Stelle des Verdauungstraktes unter der Einwirkung des Magensaftes bzw. des Darmsaftes möglichst schnell zerfallen bzw. den Wirkstoff freisetzen. Zur Einstellung der Zerfallszeit werden sogenannte Tabletten-Sprengmittel (Desintegrierungsmittel) verwendet.

An die Tabletten-Sprengmittel werden folgende Anforderungen gestellt: diese Substanzen sollen stark quellbar, kapillaraktiv, hygroskopisch und gegenüber den Wirkstoffen der Tabletten inert sein, sie dürfen aber die mechanische Festigkeit und die Verschleißfestigkeit der Tabletten nicht nachteilig beeinflussen.

Die bekannten Tabletten-Sprengmittel (Maisstärke, mikrokristalline Cellulose, durch Formaldehyd vernetztes Casein, vernetztes Polyvinylpyrrolidon usw.) entsprechen diesen Anforderungen nur teilweise. Einerseits müssen die bekannten Sprengmittel, um eine befriedigende Zerfallswirkung zu erreichen, in großen Mengen angewendet werden, was sehr kostspielig ist, andererseits führt die Anwendung der Sprengmittel in einer Menge, mit der eine gute Zerfallswirkung erzielt wird, zur Herabsetzung der mechanischen Festigkeit und zur Erhöhung der Verschleißverluste der Tabletten.

Nach der japanischen KOKAI Nr. 54 011 226 wird als Sprengmittel die Verwendung von nieder substituierter Hydroxypropylcellulose vorgeschlagen und nach der US-PS 4 086 335 wird zur Herstellung von Tabletten für die Veterinärmedizin als Sprengmittel Kitin eingesetzt.

Das Ziel der Erfindung war die Entwicklung einer Komposition und eines Verfahrens zur Herstellung von Tabletten, die die oben angegebenen Nachteile nicht aufweisen und in wäßrigem Medium rasch zerfallen, aber in trockenem Zustand die gewünschte mechanische Festigkeit haben und verschleißfest sind.

Nach dem Verfahren der Erfindung wird dieses Ziel dadurch erreicht, daß der Pulvermischung, die den Wirkstoff und übliche Formulierungshilfsstoffe enthält - gegebenenfalls vor oder nach dem Granulieren - in Wasser quellbares Cyclodextrinpolymer oder -copolymer z. B. Cyclodextrin-polyvinylalkohol-copolymer, oder Cyclodextrin-schaumpolymer in einer Menge von etwa 1 bis etwa 10 Gew.% - auf das Gewicht der Pulvermischung bezogen - zugesetzt und danach das erhaltene Gemisch in an sich bekannter Weise - gewünschtenfalls nach einem Granulieren - zu Tabletten verpreßt wird.

Die in dem Verfahren der Erfindung verwendeten Tabletten-Sprengmittel sind mit beliebigen Wirkstoffen und Zusatzstoffen kompatibel und können zur Herstellung von Tabletten beliebiger Zusammensetzung verwendet werden, sie quellen in wäßrigem Medium äußerst schnell unter Wasseraufnahme, sind gegenüber den Verdauungssäften resistent, werden aus dem Organismus in unveränderter Form, ohne absorbiert zu werden, wieder ausgeschieden und verursachen keine Toxizitätsprobleme. In den angegebenen Konzentrationen setzen sie weder die Festigkeit der Tabletten in beträchtlichem Maße herab, noch erhöhen sie die Verschleißverluste der Tabletten.

Im Gegensatz zu den bekannten Hilfsstoffen, die bislang auf diesem Anwendungsgebiet eingesetzt worden sind, bleiben in den gemäß Erfindung verwendeten Sprengmitteln die komplexbildenden Eigenschaften des anwesenden Cyclodextrins erhalten, was die Sprengmittel dazu befähigt, einen Teil des bei dem Zerfall der Tablette freigesetzten Wirkstoffes zu binden und anschließend durch eine langsame Wirkstoffabgabe eine gleichmäßige Absorption des Wirkstoffes zu ermöglichen.

Die für die Erfindung geeigneten Cyclodextrin-polymere oder -schaumpolymere können 20 bis 80 % gebundenes Cyclodextrin enthalten. Das Cyclodextrin kann entweder unsubstituiertes oder durch verschiedene Gruppen (z. B. Amino- oder Carboxy-gruppen) substituiertes $\alpha$-, $\beta$- oder $\gamma$-Cyclodextrin sein.

Zur Herstellung der gemäß Erfindung verwendeten Cyclodextrin-polymere oder -copolymere sind mehrere Verfahren bekannt (vgl. BE-PS 877 653), deren wesentliches und gemeinsames Merkmal darin besteht, das Cyclodextrin in einem alkalischen-wäßrigen Medium - gegebenenfalls in Gegenwart von Zusatzstoffen, die die physikalischen Eigenschaften des Produktes beeinflussen - mit bifunktionellen Verbindungen (z. B. Epichlorhydrin oder bis-Epoxyalkyl-äther) zu vernetzen. Bei der Herstellung von Schaumpolymeren wird das Produkt durch Zugabe von schaumbildenden Mitteln (Treibstoffen) verschäumt (HU-PA 1188 (81)).

Als Wirkstoff können beliebige, gewöhnlich in Tablettenform anwendbare Wirkstoffe eingesetzt werden.

Die Tablettierverfahren bzw. die Vorbereitung des zu tablettierenden Gemisches kann in an sich bekannter Weise erfolgen. Nach einer vorteilhaften Ausführungsform des Verfahrens der Erfindung werden der oder die Wirkstoffe und üblichen Zusatzstoffe (Füllstoffe, Verdünnungs-, Stabilisierungs-, Gleit- und Bindemittel, gegebenenfalls Aromastoffe, Farbstoffe, Substanzen zur Einstellung des pH-Wertes sowie des osmotischen

2

Druckes etc.) nach der Pulverisierung in eine homogene Pulvermischung übergeführt, danach mit etwa 1 bis etwa 10 Gew.% Cyclodextrin-polymer bzw. Cyclodextrin-schaumpolymer versetzt und die so erhaltene Mischung nach erneuter Homogenisierung mit Hilfe einer geeigneten Tablettiermaschine zu Tabletten verpreßt. Nach einer anderen Ausführungsform des Verfahrens der Erfindung wird die, den Wirkstoff und die üblichen Zusatzstoffe enthaltende Mischung zuerst in an sich bekannter Weise granuliert und dann dem so erhaltenen Granulat nach dem Trocknen etwa 1 bis etwa 70 Gew.% Cyclodextrin-polymer bzw. -schaumpolymer zugesetzt.

Bei dem Tablettieren kann beliebiger Druck angewandt werden.

Die so erhaltenen Tabletten können in an sich bekannter Weise in Dragées übergeführt werden, z. B. in dem man zunächst durch Pressen sogenannte Dragéekerne herstellt, die man dann in einem Dragéekessel mit einem gegebenenfalls gefärbten und das Dragée enterosolvent machenden Überzug oder mit einer äußeren Schutzglasur versieht.

Weitere Einzelheiten des Verfahrens der Erfindung sind den nachstehenden Beispielen zu entnehmen, ohne den Schutzumfang auf diese Beispiele einzuschränken.

**Beispiel 1**

Es werden Tabletten folgender Zusammensetzung hergestellt.

| Avicel® pH 102 | 91,6 g |
|---|---|
| Talk | 1,8 g |
| Magnesiumstearat | 1,8 g |
| Sprengmittel | 4,8 g |

"Avicel pH 102" ist eine mikrokristalline Cellulose (Hersteller: FMC Corporation, Philadelphia USA).

Als Sprengmittel werden: Maisstärke, Ultra-Amylopektin®, "Esma Spreng®" (mit Formaldehyd vernetztes Casein, Hersteller: Edelfettwerke, Werner Schlüter, Hamburg Bundesrepublik Deutschland), "Polyplasdone® XL" (vernetztes Polyvinylpyrrolidon, Hersteller: GAF Corporation, New York USA), sowie Cyclodextrin-schaumpolymer verwendet.

Die Komponenten werden in einem Becken 15 Minuten homogenisiert. Der Feuchtigkeitsgehalt der Proben beträgt 3,5 bis 4,2 % (bei 80°C innerhalb von 15 Minuten abgegebene Feuchtigkeit) und die Fließbarkeit 260 bis 370 ml/Minute (bestimmt nach der ASTM-Methode, vgl. Kedvessy: Arzneimitteltechnologie, Medicina Verlag, Budapest 1978). Das Tablettieren wird auf einer Rundläufer-Tablettiermaschine (Rotationstablettiermaschine) (Typ HORN DR 23, Hersteller: Horn) bei "mäßigem" und bei "grossem" Druck durchgeführt (diese Druckwerte sind an der Maschine einstellbare Stufen).

Durchmesser der erhaltenen Tabletten: 11 mm;
durchschnittliches Gewicht: 0,27 g.
Form: glatt-konvex;
Drehzahl der Maschine: 10/Minute;
Höhe der Tabletten: 3,4 mm bei "mäßigem Druck bzw. 3,0 mm bei "großem" Druck.

Die Kontrolltabletten enthalten anstelle des Sprengmittels "Avicel pH 102".

Die mit den verschiedenen Sprengmitteln hergestellten Tabletten wurden auf Festigkeit und Zerfallszeit untersucht. Die erhaltenen Ergebnisse sind in Tabelle I zusammengefaßt, wobei in der oberen Reihe die Werte der bei "mäßigem" Druck hergestellten Tabletten und in der unteren Reihe die Werte der bei "großem" Druck hergestellten Tabletten angegeben sind.

**Tabelle I**

| Sprengmittel/ physikalische Eigenschaften | Kontrolle | Cyclo-dextrin-schaum-polymer | Maisstärke | Ultra-Amylo-pectin | Esma Spreng | Polyplas-done XL |
|---|---|---|---|---|---|---|
| durchschnittliche Bruchfestigkeit (kp) | 6,1 | 6,9 | 4,1 | 7,2 | 6,6 | 8,0 |
| (ERWEKA) | 8,1 | 9,5 | 7,4 | 8,4 | 9,6 | 9,6 |
| Zerfallszeit (Min) | 45 | 1 | 19 | 2 | 4 | 2 |
| (USP XIX) | 65 | 9 | 60 | 57 | 38 | 3 |
| Tabletten-modul *) | 0,1 | 6,9 | 0,2 | 3,6 | 1,7 | 4,0 |
| | 0,13 | 1,05 | 0,12 | 0,15 | 0,25 | 3,2 |
| Zerfalls-koeffizient **) | 45 | 1 | 19 | 2 | 4 | 2 |
| | 7,2 | 1 | 6,7 | 6,3 | 1,2 | 0,33 |

*) Tablettenmodul $= \frac{\text{durchschnittliche Bruchfestigkeit (kp)}}{\text{durchschnittliche Zerfallszeit (Min)}}$

**) die auf die Zerfallszeit der mit Cyclodextrin-schaumpolymer hergestellten Tabletten bezogene Zerfallszeit.

**Beispiel 2**

Es werden Tabletten folgender Zusammensetzung hergestellt:

| | | | | | |
|---|---|---|---|---|---|
| Sprengmittel | 0 | 2,5 | 5,0 | 7,5 | 10 |
| Avicel pH 102 | 96,5 | 94,0 | 91,5 | 89,0 | 86,5 |
| Talk | 1,5 | 1,5 | 1,5 | 1,5 | 1,5 |
| Magnesiumstearat | 2,0 | 2,0 | 2,0 | 2,0 | 2,0 |
| | 100,0 | 100,0 | 100,0 | 100,0 | 100,0 |

Aus der Mischung werden auf einer Manesty BB-BB-Maschine Tabletten mit einem Durchmesser von 7 mm gepreßt. Die Zerfallszeit wird in Wasser bestimmt. Die gemessenen Zerfallszeiten (in Minuten) sind in Tabelle II zusammengestellt:

**Tabelle II**

| | | | | | |
|---|---|---|---|---|---|
| Sprengmittel | 0,0 | 2,5 | 5,0 | 7,5 | 10,0 |
| Cyclodextrin-schaumpolymer | | 3,3 | 0,7 | 0,5 | 0,3 |
| Maisstärke | | 25,0 | 16,6 | 13,3 | 9,3 |
| Esma Spreng | | 9,6 | 3,5 | 6,0 | 1,0 |
| Polyplasdone XL | | 6,1 | 3,5 | 0,5 | 0,5 |

**Beispiel 3**

Es werden zwei Pulvermischungen folgender Zusammensetzung hergestellt:

| | | |
|---|---|---|
| β-Cyclodextrin-schaumpolymer (Carboxygruppengehalt 1,3%) | 2,5 g | 0,0 g |
| β-Cyclodextrin-schaumpolymer (Aminogruppengehalt 1,5%) | 0,0 g | 2,5 g |
| Avicel pH 102 | 94,0 g | 94,0 g |
| Magnesiumstearat | 2,0 g | 2,0 g |
| Talk | 1,5 g | 1,5 g |
| | 100,0 g | 100,0 g |

Aus diesen Pulvermischungen werden unter einem Druck von $9,8 \cdot 10^8$ Pa Tabletten mit einem Gewicht von 0,5 g gepreßt. Die durchschnittliche Bruchfestigkeit der Tabletten beträgt mehr als 12 kp (mit einem ERWEKA-Apparat bestimmt). Die durchschnittliche Zerfallszeit der Carboxygruppen enthaltenden Tabletten beträgt 5,5 Minuten, die der Aminogruppen enthaltenden Tabletten 6,5 Minuten.

**Beispiel 4**

Es werden zwei Pulvermischungen folgender Zusammensetzung hergestellt:

| | | |
|---|---|---|
| α-Cyclodextrin-schaumpolymer (Carboxygruppengehalt 1 %) | 4,0 g | 0,0 g |
| α-Cyclodextrin-schaumpolymer (Aminogruppengehalt 1,2 %) | 0,0 g | 4,0 g |
| Avicel pH 102 | 92,0 g | 92,0 g |
| Talk | 1,5 g | 1,5 g |
| Magnesiumstearat | 2,5 g | 2,5 g |
| | 100,0 g | 100,0 g |

Aus diesen Pulvermischungen werden in der im Beispiel 3 angegebenen Weise Tabletten gepreßt. Ihre durchschnittliche Zerfallszeit in Wasser beträgt weniger als 5 Minuten.

**Beispiel 5**

Es werden zwei Pulvermischungen folgender Zusammensetzung hergestellt:

| | | |
|---|---|---|
| Tolbutamid [1-Butyl-3-4-toluol-sulfonyl)-harnstoff] | 84,0 g | 84,0 g |
| Polyvinylpyrrolidon | 3,0 g | 3,0 g |
| Cyclodextrin-schaumpolymer | 10,0 g | 0,0 g |
| Maisstärke | 0,0 g | 10,0 g |
| Talk | 1,5 g | 1,5 g |
| Magnesiumstearat | 1,5 g | 1,5 g |
| | 100,0 g | 100,0 g |

Die Pulvermischungen werden in an sich bekannter Weise mit 96 Vol.%igem Äthanol granuliert und getrocknet.

| Kennzeichen der Granulate: | | |
|---|---|---|
| Volumengewicht (g/ml) | 0,34 | 0,33 |
| Fließbarkeit (sec/100 ml) | 8,0 | 9,0 |

Die Granulate werden zu Tabletten (Gewicht 0,6 g, Durchmesser 13 mm) verpreßt.

| Kennzeichen der Tabletten: | | |
|---|---|---|
| Höhe (mm) | 4,12- 3,98 | 3,96 - 3,81 |
| durchschnittliche Festigkeit (kp) | 5,8 | 7,9 |
| Zerfallszeit (sec) | 210 | 1800 |
| Verschleißverlust (%) | 0,16 | 5,0 |
| Tablettenmodul | 1,66 | 0,26 |

Diesen Angaben ist zu entnehmen, daß die Zerfallszeit der gemäß Erfindung hergestellten Tabletten etwa achtmal kürzer als die der Kontrolltabletten und der Verschleißverlust mehr als dreißigmal geringer als der der Kontrolltabletten ist.

**Beispiel 6**

Es werden zwei Pulvermischungen folgender Zusammensetzung hergestellt:

| | | |
|---|---|---|
| Carbutamid [1-Butyl-3-sulfonyl-harnstoff] | 86,0 g | 86,0 g |
| Maisstärke | 3,6 g | 8,6 g |

Die Pulvermischungen werden mit 16 ml einer 2 %igen wäßrigen weißen Gelatinelösung granuliert, danach die erhaltenen Granulate getrocknet und mit den nachstehenden Komponenten homogenisiert:

5

| β-Cyclodextrin-schaumpolymer | 5,0 g | 0,0 g |
|---|---|---|
| Talk | 1,7 g | 1,7 g |
| Magnesiumstearat | 1,7 g | 1,7 g |

Aus dem erhaltenen Homogenisat werden Tabletten mit einem Durchmesser von 13 mm und einem Gewicht von 0,58 g hergestellt.

| Kennzeichen der Tabletten: | | |
|---|---|---|
| durchschnittliche Festigkeit (kp) | 4,5 | 4,0 |
| Zerfallszeit (sec) | 300 | 420 |
| durchschnittlicher Verschleiß- verlust (%) | 0,45 | 0,45 |
| Tablettenmodul | 0,9 | 0,57 |

**Beispiel 7**

Es werden zwei Pulvermischungen folgender Zusammensetzung hergestellt:

| Hydrochlorothiazid (6-Chlor-3,4- dihydro-2H-1,2,4-benzothiadiazin- 7-sulfonamid-1,1-dioxyd) | 19 g | 19 g |
|---|---|---|
| pulvertrockene Lactose | 60 g | 60 g |
| Avicel pH 102 | 12 g | 12 g |
| α-Cyclodextrin-schaumpolymer | 5 g | 0 g |
| Esma Spreng | 0 g | 5 g |
| Magnesiumstearat | 3 g | 3 g |
| Carbovax 6000® (Polyäthylenglykol mit einem durchschnittlichen Poly- merisationsgrad von 6000) | 1 g | 1 g |
| | 100 g | 100 g |

Die erhaltenen Mischungen werden homogenisiert und zu Tabletten mit einem Gewicht von 0,13 g und einem Durchmesser von 7 mm verpreßt.

| Kennzeichen der Tabletten: | | |
|---|---|---|
| durchschnittliche Festigkeit (kp) | 3,5 | 3,2 |
| durchschnittliche Zerfallszeit (sec) | 120 | 120 |
| durchschnittlicher Verschleiß- verlust (%) | 0,1 | 0,3 |
| Tablettenmodul | 1,75 | 1,75 |

**Beispiel 8**

Es werden zwei Pulvermischungen folgender Zusammensetzung hergestellt:

| Novamidoazofen [N-Methyl-N-(2,3- dimethyl-5-oxo-1-phenyl-3-pyrazo- lin-4-yl)-amino-methansulfonsäure] | 66,6 g | 66,6 g |
|---|---|---|
| Lactose | 23,4 g | 23,4 g |

Die Pulvermischungen werden mit einer 70 %igen ätherischen Lösung von 3,0 g Polyvinylpyrrolidon nach dem Fluidisierungssprühverfahren granuliert und die erhaltenen Granulate mit folgenden Substanzen vermischt:

| β-Cyclodextrin-blockpolymer (in Pulverform | 5,0 g | 0,0 g |
|---|---|---|
| Ultra-Amylopektin | 0,0 g | 5,0 g |
| Talk | 0,5 g | 0,5 g |
| Magnesiumstearat | 1,5 g | 1,5 g |

Die so erhaltenen Mischungen werden homogenisiert und zu Tabletten (Durchmesser 13 mm, Gewicht 750 mg) verpreßt.

Kennzeichen der Tabletten:

| durchschnittliche Festigkeit (kp) | 4,0 | 4,5 |
|---|---|---|
| durchschnittliche Zerfallszeit (sec) | 480 | 720 |
| durchschnittlicher Verschleiß- verlust (%) | 0,6 | 0,6 |
| Tablettenmodul | 0,5 | 0,36 |

**Beispiel 9**

Es werden zwei Pulvermischungen folgender Zusammensetzung hergestellt:

| Ethambutol-Dihydrochlorid [(+)-N,N'-Äthylen-bis-(2-amino-butan -1-ol)-Dihydrochlorid] | 33,3 g | 33,3 g |
|---|---|---|
| wasserfreie Lactose | 45,0 g | 45,0 g |
| Avicel pH 102 | 13,0 g | 13,0 g |
| γ-Cyclodextrin-blockpolymer (Pulver) | 6,0 g | 0,0 g |
| Esma Spreng | 0,0 g | 6,0 g |
| Carbowax 6000 (Polyäthylenglykol-mit einem durchschnittlichen Poly-merisationsgrad von 6000) | 2,0 g | 2,0 g |
| Magnesiumstearat | 0,7 g | 0,7 g |
| | 100,0 g | 100,0 g |

Die Mischungen werden homogenisiert und zu Tabletten (Durchmesser 10 mm, Gewicht 0,3 g) verpreßt.

Kennzeichen der Tabletten:

| durchschnittliche Festigkeit (kp) | 5,0 | 4,5 |
|---|---|---|
| durchschnittliche Zerfallszeit (sec) | 120 | 900 |
| durchschnittlicher Verschleiß- verlust (%) | 0,2 | 0,6 |
| Tablettenmodul | 2,5 | 0,3 |

Das in den Beispielen verwendete Cyclodextrin-blockpolymer wird nach dem Verfahren der unter der Nr. T/19 626 bekanntgemachten HU-PA CI-1845 & US-A-4 274 985 hergestellt. Bei der Herstellung der Cyclodextrinschaumpolymere wird derselbe Weg eingeschlagen mit der Ausnahme der in der HU-PA 1188/81 beschriebenen Änderungen. Am Ende der Vorpolymerisation werden nämlich dem Reaktionsgemisch kernbildende (z. B. Porzellanpulver) und schaumbildende Mittel (z. B. Distickstoffoxyd, Dichloräthan, Natriumcarbonat und Schwefelsäure etc.) zugegeben.

**Patentansprüche** für die Vertragsstaten CH, DE, FR, GB, LI.

1. Komposition zur Herstellung von in wäßrigem Medium schnell zerfallenden Tabletten,
dadurch gekennzeichnet, daß sie neben dem Wirkstoff und den üblichen Formulierungshilfsstoffen ein im Wasser quellbares Cyclodextrin-polymer, -copolymer oder Cyclodextrin-schaumpolymer in einer Menge von 1 bis 10 Gew.% enthält.

2. Verfahren zur Herstellung von in wäßrigem Medium schnell zerfallenden Tabletten durch Vermischen von Wirkstoff und üblichen Formulierungshilfsstoffen, Granulieren und Pressen,
dadurch gekennzeichnet, daß man den, den Wirkstoff und übliche Formulierungshilfsstoffe enthaltenden Pulvermischungen - gewünschtenfalls vor oder nach dem Granulieren - als Sprengmittel ein in Wasser quellbares Cyclodextrin-polymer, -copolymer oder Cyclodextrin-schaumpolymer in einer Menge von etwa 1 bis etwa 10 Gew.% - auf das Gewicht der Pulvermischung bezogen - zusetzt.

3. Verfahren nach Anspruch 2, dadurch gekennzeichnet, daß man als Cyclodextrin-copolymer ein in Wasser quellbares Cyclodextrinpolyvinyl-alkohol-copolymer verwendet.

4. Verfahren nach Anspruch 2 oder 3, dadurch gekennzeichnet, daß man als Sprengmittel ein α-Cyclodextrin, β-Cyclodextrin und/oder γ -Cyclodextrin enthaltendes Polymer, Copolymer oder Schaumpolymer verwendet.

5. Verfahren nach Anspruch 2, 3 oder 4, dadurch gekennzeichnet, daß man als Sprengmittel ein unsubstituiertes oder ein substituiertes, gegebenenfalls amino- oder carboxysubstituiertes Cyclodextrin enthaltendes Polymer, Copolymer oder Schaumpolymer verwendet.

**Patentansprüche** für den Vertragsstaat AT.

1. Verfahren zur Herstellung von in wäßrigem Medium schnell zerfallenden Tabletten durch Vermischen von Wirkstoff und üblichen Formulierungshilfsstoffen, Granulieren und Pressen, dadurch gekennzeichnet, daß man den, den Wirkstoff und übliche Formulierungshilfsstoffe enthaltenden Pulvermischungen - gewünschtenfalls vor oder nach dem Granulieren - als Sprengmittel ein in Wasser quellbares Cyclodextrin-polymer, -copolymer oder Cyclodextrin-schaumpolymer in einer Menge von etwa 1 bis etwa 10 Gew.% - auf das Gewicht der Pulvermischung bezogen - zusetzt.

2. Verfahren nach Anspruch 1, dadurch gekennzeichnet, daß man als Cyclodextrin-copolymer ein in Wasser quellbares Cyclodextrinpolyvinyl-alkohol-copolymer verwendet.

3. Verfahren nach Anspruch 1 oder 2, dadurch gekennzeichnet, daß man als Sprengmittel ein α-Cyclodextrin, β-Cyclodextrin und/oder γ-Cyclodextrin enthaltendes Polymer, Copolymer oder Schaumpolymer verwendet.

4. Verfahren nach Anspruch 1, 2 oder 3, dadurch gekennzeichnet, daß man als Sprengmittel ein unsubstituiertes oder ein substituiertes, gegebenenfalls amino- oder carboxysubstituiertes Cyclodextrin enthaltendes Polymer, Copolymer oder Schaumpolymer verwendet.

**Claims** for the contracting States: CH, DE, FR, GB, LI.

1. Composition for the preparation of tablets which rapidly decompose in an aqueous medium, characterised in that it contains, in addition to the active substance and conventional formulation excipients, a cyclodextrin polymer or copolymer or a cyclodextrin foam polymer which is capable of swelling in water in a quantity of from 1 to 10% by weight.

2. Process for preparing tablets which rapidly decompose in an aqueous medium by mixing the active substance and conventional formulation excipients, granulating and pressing, characterised in that a cyclodextrin polymer or copolymer or a cyclodextrin foam polymer capable of swelling in water is added, in a quantity of about 1 to about 10 % by weight, based on the weight of the powdered mixture, as disintegrant to the powder mixtures containing the active substance and conventional formulation excipients, if desired before or after granulation.

3. Process as claimed in claim 2, characterised in that a cyclodextrin-polyvinyl alcohol copolymer capable of swelling in water is used as the cyclodextrin copolymer.

4. Process as claimed in claim 2 or 3, characterised in that a polymer, copolymer or foam polymer containing α-cyclodextrin, β-cyclodextrin and/or γ-cyclodextrin is used as disintegrant.

5. Process as claimed in claim 2, 3 or 4, characterised in that a polymer, copolymer or foam polymer containing unsubstituted cyclodextrin or a polymer, copolymer or foam polymer containing substituted, optionally amino- or carboxy-substituted cyclodextrin, is used as disintegrant.

**Claims** for the contracting state AT.

1. Process for preparing tablets which rapidly decompose in an aqueous medium by mixing the active substance and conventional formulation excipients, granulating and pressing, characterised in that a cyclodextrin polymer or copolymer or a cyclodextrin foam polymer capable of swelling in water is added, in a quantity of about 1 to about 10 % by weight, based on the weight of the powdered mixture, as disintegrant to the powder mixtures containing the active substance and conventional formulation excipients, if desired before or after granulation.

2. Process as claimed in claim 1, characterised in that a cyclodextrin-polyvinyl alcohol copolymer capable of swelling in water is used as the cyclodextrin copolymer.

3. Process as claimed in claim 1 or 2, characterised in that a polymer, copolymer or foam polymer containing α-cyclodextrin, β-cyclodextrin and/or γ-cyclodextrin is used as disintegrant.

4. Process as claimed in claim 1, 2 or 3, characterised in that a polymer, copolymer or foam polymer containing unsubstituted cyclodextrin or a polymer, copolymer or foam polymer containing substituted,

# 0 088 098

optionally amino- or carboxy-substituted cyclodextrin, is used as disintegrant.

**Revendications** pour les Etats contractants: CH, DE, FR, GB, IT.

1. Composition pour la préparation de comprimés qui se désagrègent rapidement en milieu aqueux, caractérisée en ce qu'elle contient, en plus de la substance active et des produits auxiliaires de formulation usuels, un polymère, un copolymère ou une mousse de polymère de cyclodextrine gonflable dans l'eau, en quantité de 1 à 10 % en poids.

2. Procédé de préparation de comprimés se désagrégeant rapidement en milieu aqueux par mélange de la substance active et des produits auxiliaires de formulation usuels, mise à l'état de granulés et compression, caractérisé en ce que l'on ajoute au mélange pulvérulent contenant la substance active et des produits auxiliaires de formulation usuels - si on le désire avant ou après la mise en granulés - en tant qu'agent désagrégeant, un polymère ou copolymère de cyclodextrine ou une mousse de polymère de cyclodextrine gonflable dans l'eau, en quantité d'environ 1 à 10% en poids, par rapport au poids du mélange pulvérulent.

3. Procédé selon la revendication 2, caractérisé en ce que l'on utilise en tant que copolymère de la cyclodextrine un copolymère cyclodextrine/alcool polyvinylique gonfable dans l'eau.

4. Procédé selon la revendication 2 ou 3, caractérisé en ce que l'on utilise en tant qu'agent désagrégeant un polymère, copolymère ou une mousse de polymère contenant de l'alpha-cyclodextrine, de la bêta-cyclodextrine et/ou de la gamma-cyclodextrine.

5. Procédé selon la revendication 2, 3 ou 4, caractérisé en ce que l'on utilise en tant qu'agent désagrégeant un polymère, un copolymère ou une mousse de polymère de cyclodextrine non substituée ou substituée, éventuellement substituée par des groupes amino ou carboxy.


**Revendications** pour l'Etat contractant: AT.

1. Procédé de préparation de comprimés se désagrégeant rapidement en milieu aqueux par mélange d'une substance active et de produits auxiliaires de formulation usuels, mise à l'état de granulés et compression, caractérisé en ce que l'on ajoute au mélange pulvérulent contenant la substance active et des produits auxiliaires de formulation usuels - si on le désire avant ou après la mise en granulés -, en tant qu'agent désagrégeant, un polymère ou copolymère de cyclodextrine ou une mousse de polymère de cyclodextrine gonflable dans l'eau en quantité d'environ 1 à 10% en poids par rapport au poids du mélange pulvérulent.

2. Procédé selon la revendication 1, caractérisé en ce que l'on utilise en tant que copolymère de cyclodextrine un copolymère cyclodextrine/alcool polyvinylique gonflable dans l'eau.

3. Procédé selon la revendication 1 ou 2, caractérisé en ce que l'on utilise en tant qu'agent désagrégeant un polymère, un copolymère ou une mousse de polymère contenant de l'alpha-cyclodextrine, de la bêta-cyclodextrine et/ou de la gamma-cyclodextrine.

4. Procédé selon la revendication 1, 2 ou 3, caractérisé en ce que l'on utilise en tant qu'agent désagrégeant un polymère, un copolymère ou une mousse de polymère contenant de la cyclodextrine non substituée ou une cyclodextrine substituée, éventuellement substituée par des groupes amino ou carboxy.